# EUROPEAN PATENT APPLICATION

(11) **EP 4 582 453 A1**
(43) Date of publication of application: **09.07.2025**
(21) Application number: 23860879.8
(22) Date of filing: 30.08.2023
(51) Int. Cl.: C07K 16/28, A61P 35/00, A61K 39/00

(54) **ANTI-CTLA-4 MONOCLONAL ANTIBODY AND USE THEREOF**

(30) Priority: 02.09.2022 KR 20220111320
(71) Applicant: Cichlid Inc., Seoul 06105 (KR); Seoul National University R & DB Foundation, Seoul 08826 (KR)
(72) Inventor: LEE, Hun Seok, Seoul 06105 (KR); SHIN, Young Kee, Seoul 08826 (KR); PARK, Hee Geon, Seoul 08826 (KR); PARK, Ji Hyun, Seoul 08826 (KR)
(74) Representative: Heide, Anna Katharina
(86) International application number: PCT/KR2023/012906
(87) International publication number: WO 2024/049203

(57) **Abstract**

The present invention relates to an anti-CTLA-4 monoclonal antibody and use thereof and, more specifically, to an anti-CTLA-4 monoclonal antibody and a functional fragment thereof, and use thereof for the prevention or treatment of cancer.

## Description

The present application claims priority to Korean Patent Application No. 10-2022-0111320, filed on September 2, 2022, the entire disclosure of which is incorporated herein by reference.

The present invention relates to an anti-CTLA-4 monoclonal antibody and use thereof, and more specifically, to an anti-CTLA-4 monoclonal antibody and its functional fragments, and use thereof for the prevention or treatment of cancer.

### Background of the Invention

CTLA-4 (cytotoxic T lymphocyte-associated antigen-4, CD152) is a membrane glycoprotein expressed by activated effector T cells and is a co-inhibitory signal receptor involved in suppressing T cell proliferation, cell cycle progression, and cytokine (IL-2, IFN-γ) production by binding to the B7 ligands (CD80/CD86) of antigen-presenting cells.

Meanwhile, cancer cells, which are targeted by T cells, have a mechanism to evade attacks from T cells in the body by suppressing T cell activity through stimulation of CTLA-4 on T cells using B7 ligands. Based on this mechanism, cancer immunotherapy, developed as a third-generation anticancer agent, has been introduced.

Anticancer agents are broadly classified into first-generation chemical anticancer agents, second-generation targeted anticancer agents, and third-generation immune anticancer agents. Unlike the disadvantages of first-generation and second-generation anticancer agents, immune anticancer agents, which belong to the third generation, bind to CTLA-4, the binding site between cancer cells and T cells, and block immune evasion signals mediated by co-inhibitory signal receptors. This mechanism allows T cells, unaffected by the immune evasion mechanism of cancer cells, to destroy cancer cells. In other words, immune anticancer agents have a novel mechanism that enhances the suppressed immune function in the body against cancer cells, leading to the destruction of cancer cells. They have fewer side effects, improve the quality of life of cancer patients, and significantly extend survival periods.

Targeting CTLA-4 with antibodies is used as a therapeutic approach in various human malignancies to block the inhibitory effects mediated by CTLA-4 in T cells. Currently, anti-CTLA-4 immunemodulating monoclonal antibody therapeutics approved in Korea include ipilimumab and tremelimumab, which are used alone or in combination with other chemical therapeutics, vaccines, or other antibodies for melanoma, non-small cell lung cancer, breast cancer, prostate cancer, pancreatic cancer, hepatocellular carcinoma, and mesothelioma.

Accordingly, there is an increasing need for anti-CTLA-4 antibodies with excellent CTLA-4 binding ability that can be used as effective immune anticancer agents for cancer treatment as third-generation anticancer agents.

Accordingly, the present inventors, while conducting research to develop an antibody with specific binding affinity to human CTLA-4 protein, discovered that the antibody comprising the unique CDR sequences provided in the present invention exhibits excellent binding neutralization ability against B7-1 (CD80) and B7-2 (CD86), possesses strong ADCC (antibody-dependent cellular cytotoxicity) activity, and activates suppressed immune functions, thereby demonstrating remarkable effects in its application as a cancer therapeutic agent, and thus completed the present invention.

Therefore, an object of the present invention is to provide an antibody or a fragment thereof that binds to human CTLA-4 protein selected from the group consisting of:
i) an antibody comprising an antibody heavy chain variable region (variable region in heavy chain, V_{H}) comprising a complementarity-determining region (CDR) H1 comprising the amino acid sequence represented by SEQ ID NO: 1, a complementarity-determining region (CDR) H2 comprising the amino acid sequence represented by SEQ ID NO: 2, a complementarity-determining region (CDR) H3 comprising the amino acid sequence represented by SEQ ID NO: 3, and an antibody light chain variable region (variable region in light chain, V_{L}) comprising a complementarity-determining region (CDR) L1 comprising the amino acid sequence represented by SEQ ID NO: 4, a complementarity-determining region (CDR) L2 comprising the amino acid sequence represented by SEQ ID NO: 5, and a complementarity-determining region (CDR) L3 comprising the amino acid sequence represented by SEQ ID NO: 6;
ii) an antibody comprising an antibody heavy chain variable region (V_{H}) comprising a complementarity-determining region (CDR) H1 comprising the amino acid sequence represented by SEQ ID NO: 9, a complementarity-determining region (CDR) H2 comprising the amino acid sequence represented by SEQ ID NO: 10, a complementarity-determining region (CDR) H3 comprising the amino acid sequence represented by SEQ ID NO: 11, and an antibody light chain variable region (V_{L}) comprising a complementarity-determining region (CDR) L1 comprising the amino acid sequence represented by SEQ ID NO: 12, a complementarity-determining region (CDR) L2 comprising the amino acid sequence represented by SEQ ID NO: 13, and a complementarity-determining region (CDR) L3 comprising the amino acid sequence represented by SEQ ID NO: 14;
iii) an antibody comprising an antibody heavy chain variable region (V_{H}) comprising a complementarity-determining region (CDR) H1 comprising the amino acid sequence represented by SEQ ID NO: 17, a complementarity-determining region (CDR) H2 comprising the amino acid sequence represented by SEQ ID NO: 18, a complementarity-determining region (CDR) H3 comprising the amino acid sequence represented by SEQ ID NO: 19, and an antibody light chain variable region (V_{L}) comprising a complementarity-determining region (CDR) L1 comprising the amino acid sequence represented by SEQ ID NO: 20, a complementarity-determining region (CDR) L2 comprising the amino acid sequence represented by SEQ ID NO: 21, and a complementarity-determining region (CDR) L3 comprising the amino acid sequence represented by SEQ ID NO: 22; and
iv) an antibody comprising an antibody heavy chain variable region (V_{H}) comprising a complementarity-determining region (CDR) H1 comprising the amino acid sequence represented by SEQ ID NO: 25, a complementarity-determining region (CDR) H2 comprising the amino acid sequence represented by SEQ ID NO: 26, a complementarity-determining region (CDR) H3 comprising the amino acid sequence represented by SEQ ID NO: 27, and an antibody light chain variable region (V_{L}) comprising a complementarity-determining region (CDR) L1 comprising the amino acid sequence represented by SEQ ID NO: 28, a complementarity-determining region (CDR) L2 comprising the amino acid sequence represented by SEQ ID NO: 29, and a complementarity-determining region (CDR) L3 comprising the amino acid sequence represented by SEQ ID NO: 30.

Another object of the present invention is to provide a polynucleotide encoding the antibody or fragment thereof, a recombinant vector comprising the same, a transformed cell line comprising the same, and a method for producing the antibody or fragment thereof using the same.

Another object of the present invention is to provide a pharmaceutical composition for preventing or treating cancer comprising the antibody or fragment thereof as an active ingredient.

Another object of the present invention is to provide a pharmaceutical composition for preventing or treating cancer consisting of the antibody or fragment thereof.

Another object of the present invention is to provide a pharmaceutical composition for preventing or treating cancer consisting essentially of the antibody or fragment thereof.

Another object of the present invention is to provide the use of the antibody or fragment thereof for manufacturing a pharmaceutical composition for treating cancer.

Another object of the present invention is to provide a method for treating cancer, which comprises administering an effective amount of a pharmaceutical composition containing the antibody or a fragment thereof as an active ingredient to a subject in need thereof.

To achieve the above objectives, the present invention provides an antibody or a fragment thereof that binds to human CTLA-4 protein selected from the group consisting of:
i) an antibody comprising an antibody heavy chain variable region (V_{H}) comprising a complementarity determining region (CDR) H1 comprising the amino acid sequence represented by SEQ ID NO: 1, a complementarity determining region (CDR) H2 comprising the amino acid sequence represented by SEQ ID NO: 2, a complementarity determining region (CDR) H3 comprising the amino acid sequence represented by SEQ ID NO: 3, and an antibody light chain variable region (V_{L}) comprising a complementarity determining region (CDR) L1 comprising the amino acid sequence represented by SEQ ID NO: 4, a complementarity determining region (CDR) L2 comprising the amino acid sequence represented by SEQ ID NO: 5, and a complementarity determining region (CDR) L3 comprising the amino acid sequence represented by SEQ ID NO: 6;
ii) an antibody comprising an antibody heavy chain variable region (V_{H}) comprising a complementarity determining region (CDR) H1 comprising the amino acid sequence represented by SEQ ID NO: 9, a complementarity determining region (CDR) H2 comprising the amino acid sequence represented by SEQ ID NO: 10, a complementarity determining region (CDR) H3 comprising the amino acid sequence represented by SEQ ID NO: 11, and an antibody light chain variable region (V_{L}) comprising a complementarity determining region (CDR) L1 comprising the amino acid sequence represented by SEQ ID NO: 12, a complementarity determining region (CDR) L2 comprising the amino acid sequence represented by SEQ ID NO: 13, and a complementarity determining region (CDR) L3 comprising the amino acid sequence represented by SEQ ID NO: 14;
iii) an antibody comprising an antibody heavy chain variable region (V_{H}) comprising a complementarity determining region (CDR) H1 comprising the amino acid sequence represented by SEQ ID NO: 17, a complementarity determining region (CDR) H2 comprising the amino acid sequence represented by SEQ ID NO: 18, a complementarity determining region (CDR) H3 comprising the amino acid sequence represented by SEQ ID NO: 19, and an antibody light chain variable region (V_{L}) comprising a complementarity determining region (CDR) L1 comprising the amino acid sequence represented by SEQ ID NO: 20, a complementarity determining region (CDR) L2 comprising the amino acid sequence represented by SEQ ID NO: 21, and a complementarity determining region (CDR) L3 comprising the amino acid sequence represented by SEQ ID NO: 22; and
iv) an antibody comprising an antibody heavy chain variable region (V_{H}) comprising a complementarity determining region (CDR) H1 comprising the amino acid sequence represented by SEQ ID NO: 25, a complementarity determining region (CDR) H2 comprising the amino acid sequence represented by SEQ ID NO: 26, a complementarity determining region (CDR) H3 comprising the amino acid sequence represented by SEQ ID NO: 27, and an antibody light chain variable region (V_{L}) comprising a complementarity determining region (CDR) L1 comprising the amino acid sequence represented by SEQ ID NO: 28, a complementarity determining region (CDR) L2 comprising the amino acid sequence represented by SEQ ID NO: 29, and a complementarity determining region (CDR) L3 comprising the amino acid sequence represented by SEQ ID NO: 30.

The present invention also provides a polynucleotide encoding the antibody or fragment thereof, a recombinant vector comprising the same, a transformed cell line comprising the same, and a method for producing the antibody or fragment thereof using the same.

To achieve another objective of the present invention, the present invention provides a pharmaceutical composition for preventing or treating cancer comprising the antibody or fragment thereof as an active ingredient.

Additionally, to achieve another objective of the present invention, the present invention provides a pharmaceutical composition for preventing or treating cancer consisting of the antibody or fragment thereof.

Additionally, to achieve another objective of the present invention, the present invention provides a pharmaceutical composition for preventing or treating cancer consisting essentially of the antibody or fragment thereof.

Additionally, to achieve another objective of the present invention, the present invention provides the use of the antibody or fragment thereof for manufacturing a pharmaceutical composition for treating cancer.

Additionally, to achieve another objective of the present invention, the present invention provides a method for treating cancer comprising administering an effective amount of a pharmaceutical composition comprising the antibody or fragment thereof as an active ingredient to a subject in need thereof.

The present invention will now be described in detail.

CTLA-4 (cytotoxic T lymphocyte-associated antigen-4) has a structure similar to CD28, a T-cell surface molecule, with similar functional characteristics. Like CD28, CTLA-4 is located on band q33-q34 of human chromosome 2 and encodes a 223-amino acid protein comprising a single variable domain adjacent to two hydrophobic regions. The chromosomal sequence homology between CD28 and CTLA-4 is approximately 20%, while the amino acid sequence homology is 30% in humans.

CTLA-4, a membrane receptor of cytotoxic T cells, shares the same B7 ligands as CD28, including B7-1 (CD80) and B7-2 (CD86), and negatively affects T-cell activation. After T-cell receptor (TCR) activation, CTLA-4 is upregulated and binds to B7 with higher affinity than T-lymphocyte receptor CD28, reducing T-cell proliferation and cytokine secretion. Additionally, CTLA-4 induces reverse signaling through B7, leading to the induction of indoleamine-2, 3-dioxygenase (IDO), which causes the catabolism of tryptophan, thereby inhibiting T-cell proliferation. Furthermore, CTLA-4 is known to negatively regulate T-cell activation by promoting the expression of casitas-B-lineage lymphoma (Cbl)-b protein or inhibiting the formation of 70 kDa zeta-associated protein (ZAP 70). Recent studies have shown that CTLA-4 induces the inhibition of the PI3K/Akt pathway, cyclin D3, cyclindependent kinase (cdk4/cdk6), and nuclear transcription factor (NF-κB).

Accordingly, in the early stages of tumor formation, CTLA-4 generates inhibitory signals that weaken the immune response to tumors, thereby reducing T-cell activation and allowing cancer cells to evade T-cell attacks. Therefore, CTLA-4 plays a critical role in regulating the immune response to tumors and is considered a potential target for immuno-oncology therapies.

Thus, the present invention provides an antibody or a fragment thereof that binds to human CTLA-4 protein.

In the present invention, the term "antibody" refers to a protein, also called immunoglobulin (Ig), that selectively acts on antigens and is involved in the immune response in living organisms. Naturally occurring whole antibodies are generally composed of two pairs of polypeptides, light chains (LC) and heavy chains (HC), each consisting of several domains, or a basic unit of these LC/HC pairs. The types of heavy chains in mammalian antibodies are classified into five types, represented by the Greek letters α, β, γ, δ, and µ, which form different types of antibodies such as IgA, IgD, IgE, IgG, and IgM, depending on the type of heavy chain. The types of light chains in mammalian antibodies are classified into two types, represented by λ and κ.

The heavy and light chains of an antibody are structurally divided into variable regions and constant regions based on the variability of their amino acid sequences. The constant region of the heavy chain consists of three or four heavy chain constant regions, CH1, CH2, and CH3 (in IgA, IgD, and IgG antibodies) or CH4 (in IgE and IgM antibodies), depending on the type of antibody, while the light chain consists of one constant region, LC. The heavy and light chains are aligned side by side with their respective variable and constant regions and are connected by a single covalent disulfide bond. The variable regions of the heavy and light chains specifically bind to antigens, and the entire antibody is composed of two pairs of heavy and light chains (HC/LC), so a single antibody molecule has bivalent single specificity, binding to two identical antigens through its two variable regions. The variable region of the antibody that binds to the antigen is called the antigen-binding site, and the part of the antigen recognized by the antibody is called the epitope.

The variable region of an antibody comprising an antigen-binding site is subdivided into a framework region (FR) with low sequence variability and a hypervariable region with high sequence variability, which is a complementarity-determining region (CDR). VH and VL are each arranged in the order of FR1-CDR1-FR2-CDR2-FR3-CDR3-FR4 from the N-terminus to the C-terminus, comprising 2 CDRs and 4 FRs. Among the variable regions of the antibody, the CDR, which has the highest sequence variability, is the region that directly binds to the antigen and is most important for the antigen specificity of the antibody.

The antibody or fragment thereof according to the present invention is not limited in type as long as it has the above-described CDR, VH and VL, or the configuration of the light chain and heavy chain. The antibody may be in the form of IgG, IgA, IgM, IgE, or IgD, and particularly, it is preferably an IgG antibody. The IgG may include, but is not limited to, its subclasses such as IgG₁, IgG₂, IgG₃, and IgG₄. Furthermore, it may be a monoclonal antibody derived from a single B cell or a polyclonal antibody derived from multiple B cells, but it is preferably a monoclonal antibody, which is a population of antibodies with substantially identical amino acid sequences of the heavy chain and light chain. Additionally, the antibody or fragment thereof of the present invention may be conjugated with enzymes, fluorescent substances, radioactive substances, proteins, etc., but is not limited thereto. The antibody of the present invention may be derived from any animal, including mammals and birds, including humans, and preferably, it may be derived from humans or may be a chimeric antibody comprising a portion of an antibody derived from humans and a portion of an antibody derived from another species.

In the present invention, the fragment of the antibody refers to a fragment that retains the antigenspecific binding ability of the whole antibody, and specifically, it may be in the form of Fab, F(ab'), F(ab')₂, Fv, scFv, diabody, or dsFv.

Fab (fragment antigen-binding) is an antigen-binding fragment of an antibody, consisting of one variable domain and one constant domain of each of the heavy chain and light chain. F(ab')₂ is a fragment generated by digesting an antibody with pepsin, in which two Fabs are connected by a disulfide bond at the heavy chain hinge. F(ab') is a monomeric antibody fragment in which the heavy chain hinge is added to the Fab separated by reducing the disulfide bond of the F(ab')₂ fragment. Fv (variable fragment) is an antibody fragment consisting only of the variable regions of the heavy chain and light chain. scFv (single chain variable fragment) is a recombinant antibody fragment in which the heavy chain variable region (V_{H}) and the light chain variable region (V_{L}) are connected by a flexible peptide linker. Diabody is a fragment in which the VH and VL of scFv are connected by a very short linker, preventing them from binding to each other, and the V_{H} and V_{L} of another scFv of the same type bind to form a dimer. dsFv refers to a polypeptide in which one amino acid residue of each of V_{H} and V_{L} is substituted with a cysteine residue and is bound via an S-S bond mediated by the cysteine residues. The amino acid residue to be substituted with a cysteine residue can be selected based on the prediction of the antibody's three-dimensional structure according to known methods. In one embodiment of the present invention, the antibody or fragment thereof according to the present invention may be an antibody selected from the group consisting of: i) an antibody comprising an antibody heavy chain variable region (V_{H}) comprising a complementarity-determining region (CDR) H1 comprising the amino acid sequence represented by SEQ ID NO: 1, a complementarity-determining region (CDR) H2 comprising the amino acid sequence represented by SEQ ID NO: 2, a complementarity-determining region (CDR) H3 comprising the amino acid sequence represented by SEQ ID NO: 3, and an antibody light chain variable region (V_{L}) comprising a complementarity-determining region (CDR) L1 comprising the amino acid sequence represented by SEQ ID NO: 4, a complementarity-determining region (CDR) L2 comprising the amino acid sequence represented by SEQ ID NO: 5, and a complementarity-determining region (CDR) L3 comprising the amino acid sequence represented by SEQ ID NO: 6; ii) an antibody comprising an antibody heavy chain variable region (V_{H}) comprising a complementarity-determining region (CDR) H1 comprising the amino acid sequence represented by SEQ ID NO: 9, a complementarity-determining region (CDR) H2 comprising the amino acid sequence represented by SEQ ID NO: 10, a complementarity-determining region (CDR) H3 comprising the amino acid sequence represented by SEQ ID NO: 11, and an antibody light chain variable region (V_{L}) comprising a complementarity-determining region (CDR) L1 comprising the amino acid sequence represented by SEQ ID NO: 12, a complementarity-determining region (CDR) L2 comprising the amino acid sequence represented by SEQ ID NO: 13, and a complementarity-determining region (CDR) L3 comprising the amino acid sequence represented by SEQ ID NO: 14; iii) an antibody comprising an antibody heavy chain variable region (V_{H}) comprising a complementarity-determining region (CDR) H1 comprising the amino acid sequence represented by SEQ ID NO: 17, a complementarity-determining region (CDR) H2 comprising the amino acid sequence represented by SEQ ID NO: 18, a complementarity-determining region (CDR) H3 comprising the amino acid sequence represented by SEQ ID NO: 19, and an antibody light chain variable region (V_{L}) comprising a complementarity-determining region (CDR) L1 comprising the amino acid sequence represented by SEQ ID NO: 20, a complementarity-determining region (CDR) L2 comprising the amino acid sequence represented by SEQ ID NO: 21, and a complementarity-determining region (CDR) L3 comprising the amino acid sequence represented by SEQ ID NO: 22; and iv) an antibody comprising an antibody heavy chain variable region (V_{H}) comprising a complementarity-determining region (CDR) H1 comprising the amino acid sequence represented by SEQ ID NO: 25, a complementarity-determining region (CDR) H2 comprising the amino acid sequence represented by SEQ ID NO: 26, a complementarity-determining region (CDR) H3 comprising the amino acid sequence represented by SEQ ID NO: 27, and an antibody light chain variable region (V_{L}) comprising a complementarity-determining region (CDR) L1 comprising the amino acid sequence represented by SEQ ID NO: 28, a complementarity-determining region (CDR) L2 comprising the amino acid sequence represented by SEQ ID NO: 29, and a complementarity-determining region (CDR) L3 comprising the amino acid sequence represented by SEQ ID NO: 30.

In another embodiment of the present invention, the antibody or fragment thereof according to the present invention may preferably be an antibody selected from the group consisting of: i) an antibody comprising a heavy chain variable region comprising the amino acid sequence from the 1st to 116th of SEQ ID NO: 7 and a light chain variable region comprising the amino acid sequence from the 1st to 110th of SEQ ID NO: 8; ii) an antibody comprising a heavy chain variable region comprising the amino acid sequence from the 1st to 116th of SEQ ID NO: 15 and a light chain variable region comprising the amino acid sequence from the 1st to 109th of SEQ ID NO: 16; iii) an antibody comprising a heavy chain variable region comprising the amino acid sequence from the 1st to 121st of SEQ ID NO: 23 and a light chain variable region comprising the amino acid sequence from the 1st to 110th of SEQ ID NO: 24; and iv) an antibody comprising a heavy chain variable region comprising the amino acid sequence from the 1st to 116th of SEQ ID NO: 31 and a light chain variable region comprising the amino acid sequence from the 1st to 110th of SEQ ID NO: 32.

In yet another embodiment of the present invention, the antibody or fragment thereof according to the present invention may more preferably be an antibody selected from the group consisting of: i) an antibody comprising a heavy chain consisting of SEQ ID NO: 7 and a light chain consisting of SEQ ID NO: 8; ii) an antibody comprising a heavy chain consisting of SEQ ID NO: 15 and a light chain consisting of SEQ ID NO: 16; iii) an antibody comprising a heavy chain consisting of SEQ ID NO: 23 and a light chain consisting of SEQ ID NO: 24; and iv) an antibody comprising a heavy chain consisting of SEQ ID NO: 31 and a light chain consisting of SEQ ID NO: 32.

The present invention also provides a polynucleotide encoding the antibody or fragment thereof, a recombinant vector comprising the same, a transformed cell line comprising the same, and a method for producing the antibody or fragment thereof using the same.

In the present invention, the term 'polynucleotide' may be described as an oligonucleotide or nucleic acid, and includes DNA molecules (e.g., cDNA or genomic DNA), RNA molecules (e.g., mRNA), analogs of the DNA or RNA generated using nucleotide analogs (e.g., peptide nucleic acids and nonnaturally occurring nucleotide analogs), and hybrids thereof. The polynucleotide may be singlestranded or double-stranded. The polynucleotide refers to a nucleotide sequence encoding an antibody comprising a CDR configuration specific to human CTLA-4 protein, or a heavy chain and light chain having the configuration of VH and VL.

A polynucleotide encoding the antibody or antigen-binding fragment thereof of the present invention can be obtained by methods well known in the art. For example, based on the DNA sequence or corresponding amino acid sequence coding for a portion or the entirety of the heavy chain and light chain of the antibody, it can be synthesized using oligonucleotide synthesis techniques well known in the art, such as polymerase chain reaction (PCR).

The term 'vector' in the present invention is used for the purpose of cloning or expressing the polynucleotide of the present invention for the recombinant production of the antibody or antigen-binding fragment thereof of the present invention, and generally includes one or more of a signal sequence, origin of replication, one or more marker genes, enhancer elements, promoters, and transcription termination sequences. The vector of the present invention may preferably be an expression vector, and more preferably, a vector comprising the polynucleotide of the present invention operably linked to a regulatory sequence, such as a promoter.

A plasmid, which is a type of vector, refers to a linear or circular double-stranded DNA molecule to which external polynucleotide fragments can be attached. Other forms of vectors include viral vectors (e.g., replication-defective retroviruses, adenoviruses, and adeno-associated viruses), wherein additional DNA fragments can be introduced into the viral genome. Certain vectors are capable of autonomous replication within the host cells (e.g., bacterial vectors including bacterial origin and episomal mammalian vectors). Other vectors (e.g., non-episomal mammalian vectors) are integrated into the genome of the host cell upon introduction and are replicated along with the host genome.

In the present invention, the term 'vector' can be understood to have the same meaning as 'expression vector,' which is a type of vector capable of expressing the polynucleotide. A polynucleotide sequence is 'operably linked' to a regulatory sequence when the regulatory sequence affects the expression (e.g., level, timing, or location of expression) of the polynucleotide sequence. The regulatory sequence is a sequence that influences the expression (e.g., level, timing, or location of expression) of the nucleic acid to which it is operably linked. The regulatory sequence may exert its influence directly on the regulated nucleic acid or through the action of one or more other molecules (e.g., polypeptides binding to the regulatory sequence and/or the nucleic acid). The regulatory sequence includes promoters, enhancers, and other expression control elements.

The cell line of the present invention is not particularly limited as long as it is a cell that can be used to express the polynucleotide encoding the antibody or fragment thereof included in the expression vector of the present invention. The host cell transformed with the expression vector according to the present invention may be a prokaryote (e.g., Escherichia coli), a eukaryote (e.g., yeast or other fungi), a plant cell (e.g., tobacco or tomato plant cells), an animal cell (e.g., human cells, monkey cells, hamster cells, rat cells, mouse cells, insect cells), or a hybridoma derived from these. Preferably, it may be a cell derived from a mammal, including humans.

Prokaryotes suitable for this purpose include Gram-negative or Gram-positive organisms, such as Enterobacteriaceae, e.g., Escherichia (e.g., E. coli), Enterobacter, Erwinia, Klebsiella, Proteus, Salmonella (e.g., Salmonella typhimurium), Serratia (e.g., Serratia marcescens), and Shigella; Bacillus belonging to Bacilli (e.g., B. subtilis and B. licheniformis); Pseudomonas (e.g., P. aeruginosa); and Streptomyces. The cell of the present invention is not particularly limited as long as it can express the vector of the present invention, but preferably, it may be E. coli.

As a eukaryote, the cell of the present invention most commonly uses *Saccharomyces cerevisiae.* However, many other genera, species, and strains may be used, including but not limited to *Schizosaccharomyces pombe, Kluyveromyces* hosts such as *K. lactis, K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906), *K. thermotolerans,* and *K. marxianus; Yarrowia* (EP 402,226); *Pichia pastoris* (EP 183,070); *Candida; Trichoderma reesei* (EP 244,234); *Neurospora crassa; Schwanniomyces,* e.g., *Schwanniomyces occidentalis;* and filamentous fungi, e.g., *Neurospora, Penicillium, Tolypocladium,* and *Aspergillus* hosts, e.g., *A. nidulans* and *A. niger.*

The term 'transformation' refers to the alteration of the genotype of a host cell by the introduction of a foreign polynucleotide (a polynucleotide encoding the antibody or fragment thereof of the present invention), regardless of the method used for the transformation, and means that the foreign polynucleotide has been introduced into the host cell. The foreign polynucleotide introduced into the host cell may be maintained integrated into the genome of the host cell or maintained without integration, and the present invention includes both.

The recombinant vector capable of expressing the antibody or fragment thereof of the present invention can be introduced into cells to produce the antibody or fragment thereof by methods known in the art, including but not limited to transient transfection, microinjection, transduction, cell fusion, calcium phosphate precipitation, liposome-mediated transfection, DEAE dextran-mediated transfection, polybrene-mediated transfection, electroporation, gene gun, and other known methods for introducing nucleic acids into cells.

Additionally, the cell of the present invention may be a cultured cell that is transformed or transfected with the polynucleotide of the present invention or a vector comprising the same, which can subsequently be expressed within the host cell. A recombinant cell refers to a cell transformed or transfected with a polynucleotide to be expressed. The cell of the present invention may also include the polynucleotide of the present invention but may not express it at the desired level unless the regulatory sequence is operably linked to the polynucleotide within the cell.

The cell of the present invention can be cultured in various media. Commercially available media, such as Ham's F10 (Sigma-Aldrich Co., St. Louis, MO), Minimal Essential Medium (MEM, Sigma-Aldrich Co.), RPMI-1640 (Sigma-Aldrich Co.), and Dulbecco's Modified Eagle's Medium (DMEM, Sigma-Aldrich Co.), are suitable for culturing the cells. The medium may be supplemented, if necessary, with hormones and/or other growth factors, salts, buffers, nucleotides, antibiotics, trace elements, and glucose or equivalent energy sources.

The present invention provides a method for producing an antibody or a fragment thereof that binds to human CTLA-4 protein, comprising the steps of culturing the cell under conditions in which the polynucleotide is expressed to produce a polypeptide comprising a light chain and a heavy chain variable region, and recovering the polypeptide from the cell or the culture medium in which the cell was cultured.

In the present invention, the cell for the production method is as described above and includes a polynucleotide encoding the antibody of the present invention. The polypeptide of the production method may be the antibody or fragment thereof of the present invention itself, or it may be a form in which other amino acid sequences are additionally bound to the antibody or fragment thereof of the present invention.

In this case, the additional amino acid sequences can be removed from the antibody or fragment thereof of the present invention using methods well known to those skilled in the art. The culture conditions, including the composition of the medium and the culture conditions, may vary depending on the type of cell, and these can be appropriately selected and adjusted by those skilled in the art.

The antibody molecule may accumulate in the cytoplasm of the cell, be secreted from the cell, or be targeted to the periplasm or extracellular medium (supernatant) by an appropriate signal sequence, and it is preferable that it is targeted to the periplasm or extracellular medium. Additionally, it is preferable to refold the produced antibody molecule using methods well known to those skilled in the art to achieve a functional conformation. The recovery of the polypeptide may vary depending on the characteristics of the produced polypeptide and the cell, and this can be appropriately selected and adjusted by those skilled in the art.

The polypeptide may be produced intracellularly, in the surrounding cytoplasmic space, or directly secreted into the medium. If the polypeptide is produced intracellularly, the cell may be disrupted as a first step to release the protein. Particulate debris, host cells, or lysed fragments may be removed, for example, by centrifugation or ultrafiltration. When the antibody is secreted into the medium, the supernatant from such an expression system is typically first concentrated using commercially available protein concentration filters, such as Amicon or Millipore Pellicon ultrafiltration units. Protease inhibitors, such as PMSF, may optionally be included in preliminary steps to inhibit protein degradation, and antibiotics may be included to prevent the growth of accidental contaminants. Antibodies produced from cells may be purified, for example, using hydroxyapatite chromatography, gel electrophoresis, dialysis, and affinity chromatography, and the antibody of the present invention is preferably purified through affinity chromatography.

According to one embodiment of the present invention, the antibody of the present invention has been confirmed to exhibit excellent binding neutralization ability against B7-1 (CD80) and B7-2 (CD86), which bind to CTLA-4.

According to another embodiment of the present invention, the antibody of the present invention was confirmed to bind to HEK293 cell lines expressing CTLA-4 and to exhibit concentration-dependent and strong antibody-dependent cellular cytotoxicity (ADCC) through NK cells.

According to yet another embodiment of the present invention, the antibody of the present invention was confirmed to activate immunity in a concentration-dependent manner.

These results suggest that the antibody of the present invention specifically binds to CTLA-4, thereby activating immune cells in the body and consequently killing cancer cells. In fact, when the antibody according to the present invention was administered to mice induced with colorectal cancer, the tumor size was significantly reduced compared to the control group administered with IgG.

Therefore, the present invention also provides a pharmaceutical composition for preventing or treating cancer, comprising the antibody or fragment thereof as an active ingredient.

In the present invention, the cancer may be a solid or non-solid cancer. Solid cancer refers to cancer tumors occurring in organs such as the liver, lungs, breasts, and skin. Non-solid cancer refers to cancer occurring in the blood and is also called blood cancer. The cancer may be carcinoma, sarcoma, cancer derived from hematopoietic cells, germ cell tumor, or blastoma. The cancer may be selected from the group consisting of breast cancer, colorectal cancer, head and neck cancer, colon cancer, skin cancer, pancreatic cancer, lung cancer, gastric cancer, ovarian cancer, prostate cancer, bladder cancer, urethral cancer, liver cancer, kidney cancer, clear cell sarcoma, melanoma, brain and spinal cord tumors, brain cancer, thymoma, mesothelioma, esophageal cancer, bile duct cancer, testicular cancer, germ cell tumor, thyroid cancer, parathyroid cancer, cervical cancer, endometrial cancer, lymphoma, myelodysplastic syndromes (MDS), myelofibrosis, acute leukemia, chronic leukemia, multiple myeloma, Hodgkin's disease, endocrine cancer, and sarcoma.

The composition according to the present invention may comprise only the antibody or fragment thereof of the present invention or may be formulated in an appropriate form together with a pharmaceutically acceptable carrier and may further comprise excipients or diluents. The term "pharmaceutically acceptable" refers to a non-toxic composition that is physiologically acceptable and does not cause allergic reactions or similar responses, such as gastrointestinal disorders or dizziness, when administered to humans. The carrier may include all types of solvents, dispersion media, water-in-oil or oil-in-water emulsions, aqueous compositions, liposomes, microbeads, and microemulsions.

The pharmaceutically acceptable carrier may further include, for example, carriers for oral or parenteral administration. Carriers for oral administration may include lactose, starch, cellulose derivatives, magnesium stearate, stearic acid, and the like. Additionally, it may include various drug delivery substances used for oral administration of peptide formulations. Furthermore, carriers for parenteral administration may include water, suitable oils, saline, aqueous glucose, and glycols, and may further include stabilizers and preservatives. Suitable stabilizers include antioxidants such as sodium bisulfite, sodium sulfite, or ascorbic acid. Suitable preservatives include benzalkonium chloride, methyl- or propyl-paraben, and chlorobutanol. The pharmaceutical composition of the present invention may further comprise lubricants, wetting agents, sweeteners, flavoring agents, emulsifiers, suspending agents, and the like. Other pharmaceutically acceptable carriers and formulations may be referenced from known literature.

The composition of the present invention can be administered to mammals, including humans, by any method. For example, it can be administered orally or parenterally. Parenteral administration methods include, but are not limited to, intravenous, intramuscular, intra-arterial, intramedullary, intradural, intracardiac, transdermal, subcutaneous, intraperitoneal, intranasal, enteral, topical, sublingual, or rectal administration.

The pharmaceutical composition of the present invention can be formulated as an oral or parenteral preparation according to the administration routes described above. In the case of oral preparations, the composition of the present invention can be formulated into powders, granules, tablets, pills, sugar-coated tablets, capsules, liquid preparations, gels, syrups, slurries, suspensions, and the like using methods known in the art. For example, oral preparations can be obtained by mixing the active ingredient with a solid excipient, grinding the mixture, adding suitable auxiliaries, and processing it into a granulated mixture to produce tablets or sugar-coated tablets. Examples of suitable excipients include sugars such as lactose, dextrose, sucrose, sorbitol, mannitol, xylitol, erythritol, and maltitol; starches such as corn starch, wheat starch, rice starch, and potato starch; celluloses such as cellulose, methyl cellulose, sodium carboxymethyl cellulose, and hydroxypropyl methyl cellulose; and fillers such as gelatin and polyvinylpyrrolidone. Additionally, disintegrants such as cross-linked polyvinylpyrrolidone, agar, alginic acid, or sodium alginate may be added as needed. Furthermore, the pharmaceutical composition of the present invention may additionally include anti-coagulants, lubricants, wetting agents, flavoring agents, emulsifiers, and preservatives. In the case of parenteral preparations, the composition can be formulated into forms such as injections, creams, lotions, topical ointments, oils, moisturizers, gels, aerosols, and nasal inhalants using methods known in the art. These formulations are described in literature generally known in the field of pharmaceutical chemistry.

The total effective amount of the composition of the present invention can be administered to a patient as a single dose or through a fractionated treatment protocol involving multiple doses over an extended period. The pharmaceutical composition of the present invention may vary in the content of the active ingredient depending on the severity of the disease. Preferably, the total daily dose of the pharmaceutical composition of the present invention is about 0.01 µg to 10,000 mg per kg of patient body weight, more preferably 0.1 µg to 500 mg. However, the dosage of the pharmaceutical composition is determined by considering various factors such as the formulation method, administration route, frequency of treatment, as well as the patient's age, weight, health condition, gender, severity of the disease, diet, and excretion rate. Therefore, one skilled in the art can determine the appropriate effective dosage of the composition of the present invention by taking these factors into account. The pharmaceutical composition according to the present invention is not particularly limited in its formulation, administration route, or method of administration as long as it exhibits the effects of the present invention.

Additionally, the present invention provides the use of the antibody or a fragment thereof for manufacturing a pharmaceutical composition for treating cancer.

Furthermore, the present invention provides a method for treating cancer, comprising administering an effective amount of a pharmaceutical composition comprising the antibody or a fragment thereof as an active ingredient to a subject in need thereof.

The term "effective amount" in the present invention refers to an amount that, when administered to a subject, exhibits effects such as improvement, treatment, detection, diagnosis, inhibition, or reduction of cancer or the disease. The term "subject" may include animals, preferably mammals, particularly humans, and may also include cells, tissues, or organs derived from animals. The subject may be a patient in need of the aforementioned effects.

The term "treatment" in the present invention comprehensively refers to improving symptoms caused by cancer or the disease, and may include curing the disease, substantially preventing it, or improving the condition. It may also include alleviating, curing, or preventing one or more symptoms or most symptoms caused by the disease, but is not limited thereto.

The term "comprising" as used herein is synonymous with "including" or "characterized by" and does not exclude additional components or steps not specifically mentioned in the composition or method according to the present invention. The term "consisting of" means excluding additional elements, steps, or components not specifically described. The term "consisting essentially of" means that the composition or method may include substances or steps that do not substantially affect the basic characteristics of the described substances or steps.

Accordingly, the present invention provides an antibody or a fragment thereof that binds to human CTLA-4 protein. The antibody of the present invention binds to CTLA-4 of T cells, thereby inhibiting the stimulation of CTLA-4 caused by cancer cells, preventing the inactivation of T cells, and enabling T cells to attack cancer cells. At the same time, it can exert a strong ADCC effect through NK cells, thereby exhibiting remarkable effects as an anticancer agent.

### Brief Description of the Drawings

Figure 1 shows that the top 4 clones among the scFv sequences obtained through phage display were converted into the form of Full IgG1, and the clones were expressed and purified as confirmed by SDS-PAGE.
Figure 2 shows the results of the binding ability of the anti-CTLA-4 antibody using a flow cytometer with the HEK293 cell line.
Figure 3 shows the results of a Western blot using the HEK293 cell line overexpressing human CTLA-4 protein and the anti-CTLA-4 antibody.
Figure 4 shows the results of immunoprecipitation (IP) using the HEK293 cell line overexpressing human CTLA-4 protein and the anti-CTLA-4 antibody.
Figure 5 shows the results of the binding neutralization ability of the anti-CTLA-4 antibody to B7-1 (CD80) and B7-2 (CD86) through competitive ELISA.
Figure 6 shows the results of an ADCC assay using the NK92MI-CD16 cell line on the HEK293 cell line overexpressing human CTLA-4 protein.
Figure 7 shows the results of immune activation through the anti-CTLA-4 antibody using ELISA after stimulating human peripheral blood mononuclear cells with Staphylococcal enterotoxin B (SEB).
Figure 8 shows the results of immune activation through the anti-CTLA-4 antibody using ELISA after conducting a mixed lymphocyte reaction with human peripheral blood mononuclear cells from different donors.
Figure 9 shows the results of the *in vivo* anti-cancer efficacy of the anti-CTLA-4 antibody using a mouse model introduced with human CTLA-4 protein.

Hereinafter, the present invention will be described in detail.

However, the following examples are merely illustrative of the present invention, and the scope of the present invention is not limited to the following examples.

### Example 1. Screening of scFv specifically binding to human CTLA-4 protein

### 1-1. Antigen and scFv phage selection

CTLA-4 as an antigen was provided in the form of a recombinant protein (hCTLA4-Fc, RND systems, Cat. No 325-CT) during the screening process. To screen for antibodies specifically binding to CTLA-4, the phage display method (phage library display) was used. The library used was a synthetic human scFv library, and specific information about the library is described in publicly known literature in the art. The scFv expressed from the scFv library was tagged with an HA tag to allow detection by anti-HA FITC antibody (Genscript, A01621). Using the scFv library, biopanning was performed as follows. The antigen hCTLA4-Fc, RND systems, Cat. No 325-CT) was coated by adding 1 mL at a concentration of 10 µg/mL to an immunotube and leaving it at 200 rpm, 4°C for more than 12 hours. The coated immunotube was washed three times with 0.05% PBS-T and blocked with 1 mL of 3% BSA blocking buffer at room temperature for 2 hours. After 2 hours, it was washed three times with 0.05% PBS-T. The blocked scFv library stock was added to the immunotube coated with the antigen and incubated at 200 rpm, 37°C for 1 hour. The unbound scFv library stock was discarded, and the tube was washed three times with 0.05% PBS-T. To elute specifically bound scFv-phages, they were reacted with 100 mM TEA (triethylamine) at room temperature for 5 minutes, neutralized with pH 8.5 Tris, and prepared in the form of scFv-antigen conjugates. The prepared scFv-antigen conjugates were added to E. coli ER2537 cells for infection, and cultured overnight at 37°C on LB/ampicillin/glucose agar medium. The E. coli ER2537 cells were transferred to SB/ampicillin medium and cultured until the OD600 value reached 0.5, after which 1x10¹¹~1x10¹² helper phages were added and cultured again at 37°C for 1 hour. Kanamycin was then added, and the culture was incubated overnight. The overnight culture was centrifuged, and the supernatant was reacted with PEG solution at 4°C, followed by centrifugation to separate the pellet. The pellet was dissolved in PBS, centrifuged, and the obtained supernatant was secured as the scFv library solution. This process was repeated four times to secure scFv candidates specifically binding to the CTLA-4 antigen.

### 1-2. Selection of scFv antibodies specifically binding to CTLA-4

To select scFv with excellent binding ability from the scFv candidates obtained in Example 1-1, ELISA analysis was performed on CTLA-4 expressing cell lines. The CTLA-4 expressing cell line was prepared by transfecting a human CTLA-4 expression vector into HEK293 cells and selecting transformants treated with 50 µg/mL of Hygromycin B. Each library stock at the end of each panning step in Example 1-1 was cultured overnight on SB/ampicillin/glucose agar medium, and each single colony was inoculated into 200 µL of SB/ampicillin medium and cultured at 37°C for 3 hours. Subsequently, IPTG was added to a final concentration of 1 mM, and the culture was incubated again at 30°C overnight. After the culture was completed, the culture medium was centrifuged to separate the cells, which were then lysed using TES buffer to obtain scFv. The obtained scFv was treated on plates where CTLA-4 cells were dispensed at 1x10⁵ per well and reacted at room temperature for 1 hour. Then, a secondary antibody (anti-HA HRP, Santa Cruz, Cat. NO. sc-7392) was added and reacted for 40 minutes. After the secondary antibody reaction was completed, TMB was added to induce a color reaction, and the results were analyzed using an ELISA reader (450 nm). By relatively comparing the ELISA analysis values, the top 4 excellent scFv were selected (3E4, 4B6, 4F2, 4G1). The amino acid sequences of the selected scFv are shown in Table 1 below.

**Table 1**

| Antibody | | | Amino acid sequence | SEQ ID NO. |
|---|---|---|---|---|
| 3E4 | Heavy chain variable region | CDR1 | NYAMS | SEQ ID NO.1 |
| | | CDR2 | VIYYDGSNK | SEQ ID NO.2 |
| | | CDR3 | ARNTYRFDY | SEQ ID NO.3 |
| | Light chain variable region | CDR1 | SGSSSNIGNNAVT | SEQ ID NO.4 |
| | | CDR2 | SDSNRPS | SEQ ID NO.5 |
| | | CDR3 | GSWDSSLSG | SEQ ID NO.6 |
| 4B6 | Heavy chain variable region | CDR1 | GYSMS | SEQ ID NO.9 |
| | | CDR2 | SISPSGGNT | SEQ ID NO.10 |
| | | CDR3 | AKTSELFDY | SEQ ID NO.11 |
| | Light chain variable region | CDR1 | TGSSSNIGSNDVN | SEQ ID NO.12 |
| | | CDR2 | NDSHRPS | SEQ ID NO.13 |
| | | CDR3 | GTWDYSLSANDSHRPS | SEQ ID NO.14 |
| 4F2 | Heavy chain variable region | CDR1 | SYDMS | SEQ ID NO.17 |
| | | CDR2 | SIYPDNSTI | SEQ ID NO.18 |
| | | CDR3 | AKSTGLFDY | SEQ ID NO.19 |
| | Light chain variable region | CDR1 | TGSSSNIGSNYVN | SEQ ID NO.20 |
| | | CDR2 | ADSQRQR | SEQ ID NO.21 |
| | | CDR3 | GSWDDSLNA | SEQ ID NO.22 |
| 4G1 | Heavy chain variable region | CDR1 | GYDMS | SEQ ID NO.25 |
| | | CDR2 | GISPGTGST | SEQ ID NO.26 |
| | | CDR3 | ARDPTVRTHLWFDY | SEQ ID NO.27 |
| | Light chain variable region | CDR1 | SGSSSNIGNNYVT | SEQ ID NO.28 |
| | | CDR2 | ADSHRPS | SEQ ID NO.29 |
| | | CDR3 | GAWDDSLSG | SEQ ID NO.30 |

### Example 2. Conversion, expression, and purification of scFv antibodies into IgG 2-1. Preparation of full IgG expression vector

The selected scFv was converted into the form of IgG, a more commonly used antibody. Based on the CDR region of the scFv, an expression vector capable of expressing the full IgG form was prepared. First, the light chain variable region and heavy chain variable region of the scFv were obtained through PCR, using the primers shown in Table 2 below. The light chain variable region sequence was cloned from the expression vector pcDNA 3.3 (Invitrogen), which contains the light chain constant region sequence, and the heavy chain variable region sequence was cloned from the expression vector pOptiVEC (Invitrogen), which contains the heavy chain constant region sequence. Together with the light chain and heavy chain constant regions cloned from the vectors, the light chain variable region and heavy chain variable region of the scFv were expressed, resulting in the production of a full IgG antibody containing the CDR region of the scFv.

**Table 2**

| Antibody | Variable region | Primer sequence | SEQ ID NO. |
|---|---|---|---|
| 3E4 | Light chain F | ATT CGA TCG ATA TGG AGA CAG ACA CAC TCC TGC TAT GGG TAC TGC TGC TCT GGG TTC CAG GTT CCA CGT GGC AGT CTG TGC TGA CTC AGC CA | SEQ ID NO. 33 |
| | Light chain R | AGC CAC CGT ACG TAG GAC CGT CAG CTT GGT GCC | SEQ ID NO. 34 |
| | Heavy chain F | ATT CGA TCG ATA TGG AGA CAG ACA CAC TCC TGC TAT GGG TAC TGC TGC TCT GGG TTC CAG GTT CCA CGT GGG AGG TGC AGC TGT TGG AGT CT | SEQ ID NO. 35 |
| | Heavy chain R | CTT GGT GCT AGC TGA GCT CAC GGT GAC CAG TGT | SEQ ID NO. 36 |
| 4B6 | Light chain F | ATT CGA TCG ATA TGG AGA CAG ACA CAC TCC TGC TAT GGG TAC TGC TGC TCT GGG TTC CAG | SEQ ID NO. 37 |
| | | GTT CCA CGT GGC AGT CTG TGC TGA CTC AGC CA | |
| | Light chain R | AGC CAC CGT ACG TAG GAC CGT CAG CTT GGT GCC | SEQ ID NO. 38 |
| | Heavy chain F | ATT CGA TCG ATA TGG AGA CAG ACA CAC TCC TGC TAT GGG TAC TGC TGC TCT GGG TTC CAG GTT CCA CGT GGG AGG TGC AGC TGT TGG AGT CT | SEQ ID NO. 39 |
| | Heavy chain R | CTT GGT GCT AGC TGA GCT CAC GGT GAC CAG TGT | SEQ ID NO. 40 |
| 4F2 | Light chain F | ATT CGA TCG ATA TGG AGA CAG ACA CAC TCC TGC TAT GGG TAC TGC TGC TCT GGG TTC CAG GTT CCA CGT GGC AGT CTG TGC TGA CTC AGC CA | SEQ ID NO. 41 |
| | Light chain R | AGC CAC CGT ACG TAG GAC CGT CAG CTT GGT GCC | SEQ ID NO. 42 |
| | Heavy chain F | ATT CGA TCG ATA TGG AGA CAG ACA CAC TCC TGC TAT GGG TAC TGC TGC TCT GGG TTC CAG GTT CCA CGT GGG AGG TGC AGC TGT TGG AGT CT | SEQ ID NO. 43 |
| | Heavy chain R | CTT GGT GCT AGC TGA GCT CAC GGT GAC CAG TGT | SEQ ID NO. 44 |
| 4G1 | Light chain F | ATT CGA TCG ATA TGG AGA CAG ACA CAC TCC TGC TAT GGG TAC TGC TGC TCT GGG TTC CAG GTT CCA CGT GGC AGT CTG TGC TGA CTC AGC CA | SEQ ID NO. 45 |
| | Light chain R | AGC CAC CGT ACG TAG GAC CGT CAG CTT GGT GCC | SEQ ID NO. 46 |
| | Heavy chain F | ATT CGA TCG ATA TGG AGA CAG ACA CAC TCC TGC TAT GGG TAC TGC TGC TCT GGG TTC CAG GTT CCA CGT GGG AGG TGC AGC TGT TGG AGT CT | SEQ ID NO. 47 |
| | Heavy chain R | CTT GGT GCT AGC TGA GCT CAC GGT GAC CAG TGT | SEQ ID NO. 48 |

### 2-2. Preparation of full IgG antibody expressing cell line

Using CHO-S cells (Life Technologies Inc.), IgG antibody expressing cell lines were prepared. The gene sequences encoding the heavy and light chains obtained in Example 2-1 were codon-optimized for Cricetulus griseus species and cloned into the Freedom^{®} pCHO1.0 Vector, followed by transfection into CHO-S cells using a transfection reagent (FreeStyle^{™} MAX Reagent; Life Technologies Inc.). To select antibody-expressing cell lines after transfection, a two-step selection process was performed using puromycin and MTX (methotrexate). Specifically, in the first selection, puromycin 10 µg/mL, MTX 100 nM, or puromycin 20 µg/mL, MTX 200 nM were used, and when the cell survival rate met the criteria, the second process was conducted. In the second selection, puromycin 30 µg/mL, MTX 500 nM, or puromycin 50 µg/mL, MTX 1000 nM were used, and when the final cell survival rate met the criteria, the second selection was completed, and groups with high expression levels were selected through SFB (Simple Fed Batch).

### 2-3. Production and purification of full IgG antibody

Each antibody-producing cell line prepared in Example 2-2 was cultured in CD FortiCHO^{™} medium under conditions of 8% CO₂, 37°C, 100~120 rpm, with glucose added at 4 g/L, 4 g/L, and 6 g/L on days 3, 5, and 7, respectively, for a total of 14 days. After the culture was completed, the culture medium was centrifuged at 6000g using an ultra-centrifuge, and the supernatant was filtered using a 0.2 µm filter. For purification, Protein A resin (Mabselect SuRe, 11-0026-01 AD, GE Healthcare Life Sciences) was used, and equilibrium buffer (20 mM Sodium Phosphate, 150 mM NaCl, pH 7.2), wash buffer (35 mM Sodium Phosphate, 500 mM NaCl, pH 7.2), and elution buffer (0.1 M Sodium Citrate, pH 3.6) were used. Using AKTA^{™} avant, the equilibrium buffer was applied at twice the column volume, the wash buffer at five times the column volume, and the elution buffer at five times the column volume for purification, and pH 8.0 Tris-HCl solution was added at 1/5 volume for neutralization during elution. After two buffer exchanges with PBS using a filtration membrane (CelluSep, 1430-45), the solution was concentrated using a centrifugal filter (Amicon Ultra-15, UFC905024, Merck).

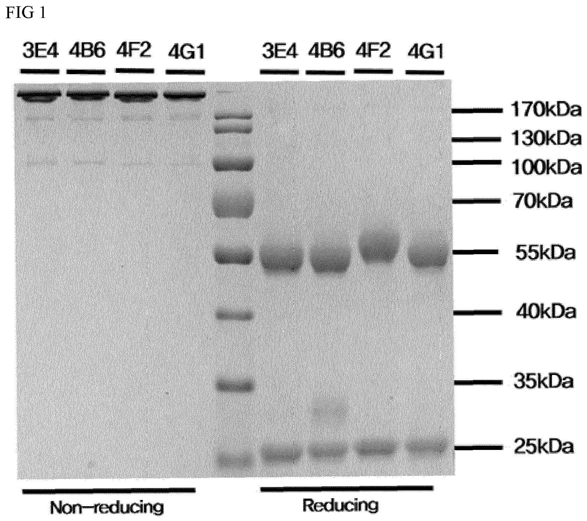

The IgG antibody proteins produced under both reducing and non-reducing conditions were confirmed using conventional SDS-PAGE techniques, and it was verified that the light and heavy chains of each antibody were well expressed at the expected molecular weights. Figure 1 shows the SDS-PAGE confirmation results for each IgG antibody.

### Example 3. Evaluation of binding specificity and binding strength of the antibody to CTLA-4 3-1. Preparation of CTLA-4/HEK293 cell line

To confirm the antigen specificity of the antibodies prepared in Example 2-3, the binding to human CTLA-4 protein was verified.

The gene encoding CTLA-4 was cloned into pCMV3-C-FLAG (Sino Biological). Each of the CTLA-4 expression vectors thus produced was transfected into HEK293 cells using the Fugene HD (E231A, Promega) transfection reagent, and resistant cell lines were selected using Hygromycin B.

### 3-2. Evaluation of the binding ability of the antibody of the present invention to CTLA-4/HEK293 cell line

For the CTLA-4/HEK293 cell line produced in Example 3-1, the cross-reactivity of the antibody prepared in Example 2-3 was confirmed. Naive HEK293 cells were used as a negative control. First, the cells were dissociated into single cells using a cell dissociation buffer (Gibco, 13151-014), seeded at 2.5x10⁵ cells per well, and reacted with each antibody at 10 µg/ml on ice for 1 hour. After the reaction, the cells were washed with 1% FBS/PBS and treated with a secondary antibody, mouse anti-human IgG-PE (366904, Biolegend), at a 1:100 dilution, followed by a reaction on ice for 1 hour. After the reaction, the cells were washed with 1% FBS/PBS and analyzed using a flow cytometer, BD FACS Lyrics.

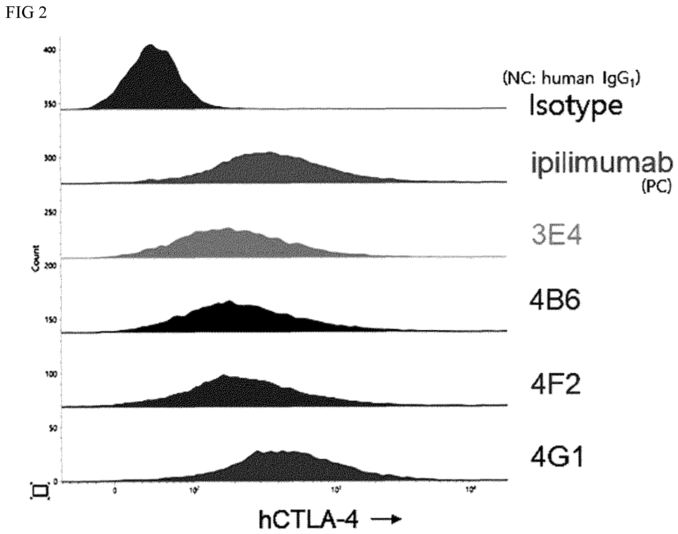

Figure 2 shows the results of the flow cytometry analysis, which comparatively confirm the binding ability of the antibody to CTLA-4.

### 3-3. Confirmation of CTLA-4-specific binding - western blot

For the CTLA-4/HEK293 cell line produced in Example 3-1, the ability of the antibody prepared in Example 2-3 to recognize denatured CTLA-4 antigens was confirmed. Naive HEK293 cells were used as a negative control. First, the cells were dissociated into single cells using a cell dissociation buffer, lysed with RIPA buffer (BIOSESANG) containing protease inhibitors and phosphatase inhibitors at 200 rpm, 4°C for 1 hour, and centrifuged at 15,000 rpm, 4°C for 15 minutes to collect the supernatant. The dissolved proteins were quantified using a BCA assay (ThermoFisher Scientific). The quantified proteins were reduced by adding 5X sample loading buffer (BIOSESANG) containing DTT and boiling for 10 minutes. Equal amounts of protein were loaded onto SDS-PAGE, separated by size, transferred to a PVDF membrane, and blocked with 5% skim milk at 4°C for 1 hour. The antibody prepared in Example 2-3 was used as the primary antibody and incubated with the PVDF membrane overnight. After washing three times with 0.05% TBS-T, the membrane was treated with a secondary antibody, anti-human Fc HRP antibody (Genscript), at a 1:2000 dilution and incubated for 30 minutes. After washing three times with 0.05% TBS-T, ECL solution (Bio-rad) was added, and the membrane was developed on film in a darkroom.

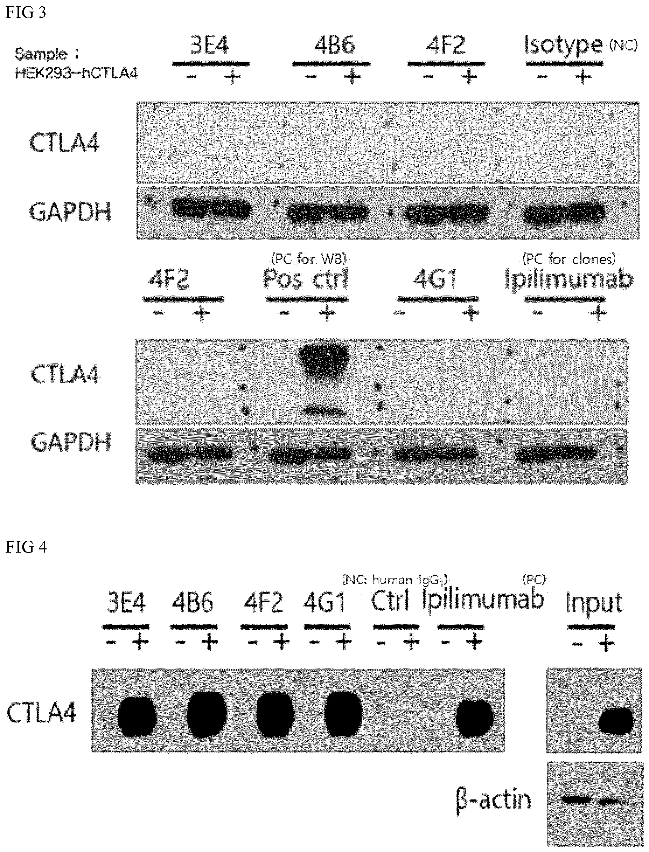

As shown in Figure 3, it was confirmed that the antibody did not recognize the denatured CTLA-4 antigen.

### 3-4. Confirmation of CTLA-4-specific binding - immunoprecipitation

For the CTLA-4/HEK293 cell line produced in Example 3-1, the ability of the antibody prepared in Example 2-3 to recognize native CTLA-4 antigens was confirmed. Naive HEK293 cells were used as a negative control. The cells were dissociated using a scraper and resuspended in PBS containing protease inhibitors and phosphatase inhibitors. The cells were physically lysed using a sonicator, and the proteins were quantified using a BCA assay. The quantified proteins were incubated with the antibody for 1 hour, while Protein A beads (Sigma) were blocked with 5% skim milk for 1 hour. The protein-antibody solution was then incubated with the blocked beads for 1 hour and washed three times with PBS. The proteins and antibodies bound to the Protein A beads were separated and reduced by adding 5X sample loading buffer containing DTT and reacting at 70°C for 10 minutes. The samples were analyzed by western blot using an anti-human CTLA-4 antibody (Cell Signaling Technology) to confirm whether the antibody recognized native CTLA-4 antigens.

As shown in Figure 4, it was confirmed that the antibody specifically binds to native CTLA-4 antigens.

### Example 4. Confirmation of binding neutralization ability to B7-1 (CD80) and B7-2 (CD86)

To confirm the binding neutralization ability of the antibody prepared in Example 2-3, the CTLA4-B7-1 (CD80) and CTLA4-B7-2 (CD86) binding neutralization ability was evaluated using ELISA. Human CTLA-4 protein (hCTLA4-Fc, RND Systems, Cat. No 325-CT) was coated at 250 ng per well on a 96-well plate and incubated overnight at 4°C. The plate was washed three times with 0.1% PBS-T and blocked with 50 mg/mL BSA/PBS at room temperature for 2 hours. The blocked 96-well plate was washed three times with 0.1% PBS-T, and the anti-CTLA-4 antibody was diluted from 60 µg/ml in a 1/3 dilution series and added. B7-1 (CD80)-biotin and B7-2 (CD86)-biotin (Sino Biological) were added and incubated at room temperature for 2 hours. Ipilimumab (BMS) was used as a positive control in the same amount. After binding, the plate was washed three times with 0.1% PBS-T, treated with Streptavidin-HRP (R&D Systems) at a 1:5000 dilution, and incubated at room temperature for 1 hour. After incubation, the plate was washed three times with 0.1% PBS-T, and 100 µL of TMB solution (Surmodics) was added to each well and reacted at room temperature for 20 minutes. Stop solution was added at 50 µL per well, and the absorbance at 450 nm was measured using an ELISA reader.

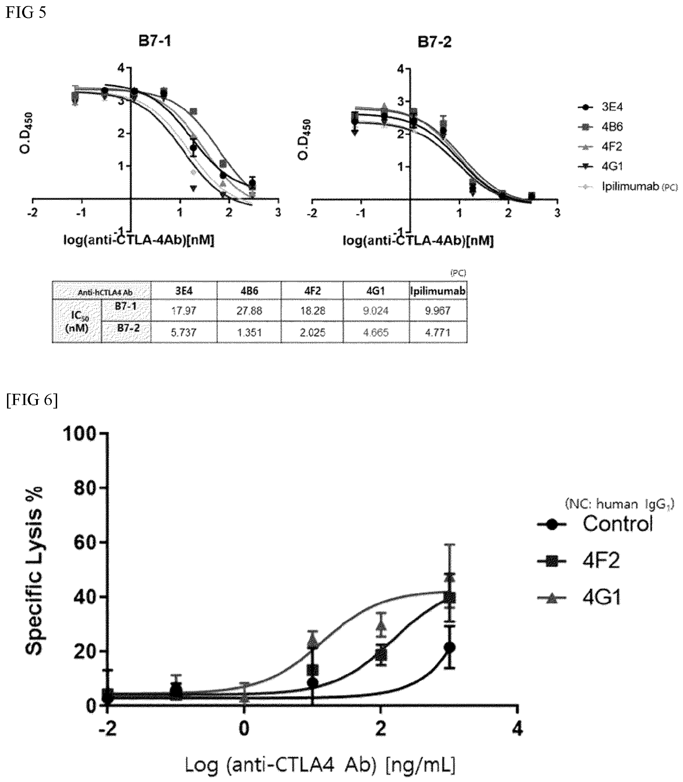

As shown in Figure 5, it was confirmed that the antibody inhibited the binding of CTLA4-B7-1 (CD80) and CTLA4-B7-2 (CD86).

### Example 5. Confirmation of antibody-dependent cytotoxicity

For the CTLA-4/HEK293 cell line produced in Example 3-1, the antibody-dependent cytotoxicity of the antibody prepared in Example 2-3 was confirmed. The CTLA-4/HEK293 cell line was used as the target cell, and a cell line transfected with human CD16 into NK92MI cells was used as the effector cell. The target cells were dissociated into single cells using a dissociation buffer, seeded at 2.0x10⁴ cells per well in a 96-well plate, and incubated overnight. The effector cells were added at 1.0x10⁵ cells per well, and the anti-CTLA-4 antibody was diluted from 1 µg/ml in a 1/10 dilution series and added to the target cells. The cells were incubated at 37°C for 4 hours. Then, 50 µL of the supernatant was used to perform an LDH assay (Promega, CytoTox 96^{®}) to confirm cytotoxicity. As shown in Figure 6, it was confirmed that the antibody induced concentration-dependent cell death of CTLA-4-expressing cells through antibody-dependent cytotoxicity mediated by NK cells.

### Example 6. Confirmation of immune activation ability

### 6-1. Immune activation by anti-CTLA-4 antibody (IL-2)

To confirm the immune activation ability of the antibody prepared in Example 2-3, a Staphylococcal enterotoxin B (SEB) activation assay was performed using human PBMCs. Peripheral blood concentrated in an LRS chamber was diluted with 2% FBS/PBS. Histopaque^{®}-1077 (Sigma) was added at 25 mL to a 50 mL tube, and 25 mL of diluted peripheral blood was gently layered on top, followed by centrifugation at 1200g for 10 minutes. After centrifugation, the supernatant was removed, and the human peripheral blood mononuclear cell (PBMC) layer was collected and transferred to a new 50 mL tube. The PBMCs were washed three times with 2% FBS/PBS washing buffer at 300g for 8 minutes to obtain PBMCs. The isolated human PBMCs were seeded at 2.0x10⁵ cells per well in a 96-well plate, and SEB (Abion) at 100 ng/mL and the anti-CTLA-4 antibody diluted from 30 µg/ml in a 1/3 dilution series were added to the cell culture medium and incubated at 37°C for 3 days. After 3 days, 100 µL of the supernatant was used for subsequent analysis. The amount of IL-2 secretion through immune activation was measured using ELISA (Human IL-2 DuoSet ELISA, R&D Systems, Cat. No. DY202).

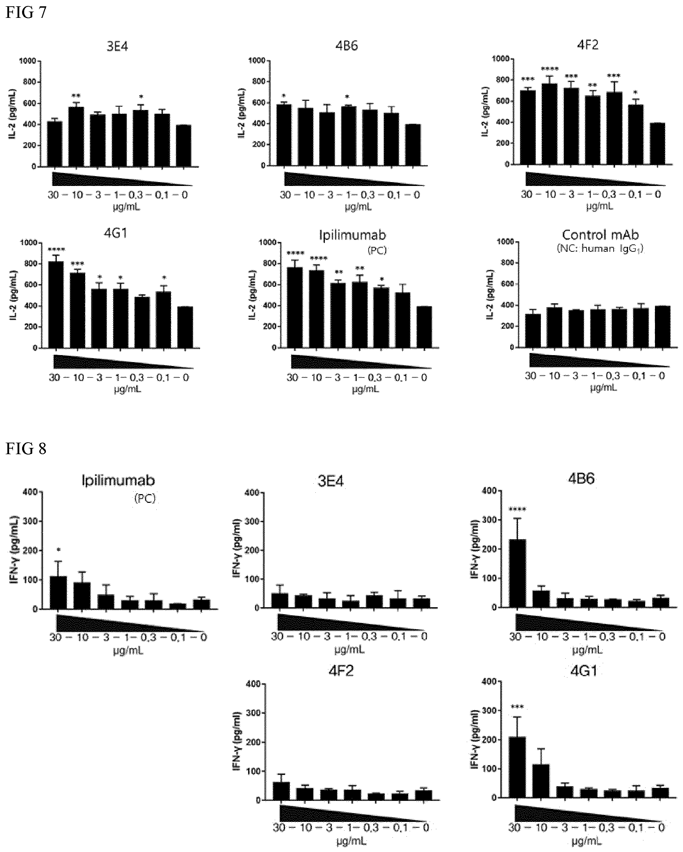

As shown in Figure 7, it was confirmed that the antibody maximized IL-2 secretion from human PBMCs through immune activation.

### 6-2. Confirmation of immune activation by anti-CTLA-4 antibody through mixed lymphocyte reaction (MLR)

To confirm the immune activation ability of the antibody prepared in Example 2-3, a mixed lymphocyte reaction (MLR) assay was conducted using PBMCs from different donors. The PBMCs to be used as stimulators were treated with mitomycin C to inhibit cell proliferation through the human PBMCs isolated in Example 6-1. PBMCs from another donor to be used as responders and PBMCs to be used as stimulators were each added at 1.0x10⁵ cells per well in a 96-well plate, and the anti-CTLA-4 antibody was diluted to a concentration of 30 µg/ml in a 1/3 serial dilution and treated. The plate was incubated at 37°C for 5 days. After 5 days, 100 µl of the supernatant was collected for subsequent analysis. The amount of IFN-γ secretion through immune activation was measured using ELISA (Human IFN-gamma DuoSet ELISA, R&D systems, Cat. No. DY285B). As a result, as shown in Figure 8, it was confirmed that the antibody increased the secretion of IFN-γ in a concentration-dependent manner.

### Example 7. Confirmation of in vivo anti-cancer efficacy of the antibody

To confirm the *in vivo* anti-cancer efficacy of the anti-CTLA-4 antibody, the prepared anti-CTLA-4 antibody was injected into human CTLA-4-introduced mice induced with cancer, and changes in tumor size were observed.

First, 5x10⁵ cells of the mouse colon cancer cell line MC38 were subcutaneously injected into human CTLA-4 protein-introduced mice. When the average tumor size reached 150 mm³, the mice were randomly assigned to control and antibody treatment groups with 5 mice per group. The control group was administered IgG, and the antibody treatment group was administered the 4G1 antibody at a dose of 20 mg/kg three times a week for a total of 2 weeks. Tumor size was measured using calipers and calculated as long axis x (short axis)² mm³.

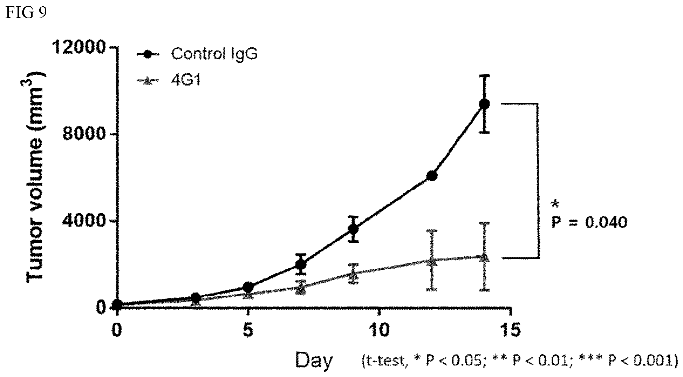

As a result, as shown in Figure 9, it was confirmed that the tumor size in the 4G1 antibody treatment group was statistically significantly reduced compared to the control group (t-test, p value=0.04).

As described above, the present invention provides an antibody or a fragment thereof that binds to human CTLA-4 protein. The antibody of the present invention binds to CTLA-4 of T cells, thereby inhibiting the stimulation of CTLA-4 caused by cancer cells, preventing the inactivation of T cells, and enabling T cells to attack cancer cells. At the same time, it can exert a strong ADCC effect through NK cells, showing remarkable efficacy as an anticancer agent, and thus has high industrial applicability.

## Claims

1. An antibody or a fragment thereof that binds to human CTLA-4 protein selected from the group consisting of:
i) an antibody comprising an antibody heavy chain variable region (V_{H}) comprising a complementarity determining region (CDR) H1 comprising the amino acid sequence represented by SEQ ID NO: 1, a complementarity determining region (CDR) H2 comprising the amino acid sequence represented by SEQ ID NO: 2, a complementarity determining region (CDR) H3 comprising the amino acid sequence represented by SEQ ID NO: 3, and an antibody light chain variable region (V_{L}) comprising a complementarity determining region (CDR) L1 comprising the amino acid sequence represented by SEQ ID NO: 4, a complementarity determining region (CDR) L2 comprising the amino acid sequence represented by SEQ ID NO: 5, and a complementarity determining region (CDR) L3 comprising the amino acid sequence represented by SEQ ID NO: 6;
ii) an antibody comprising an antibody heavy chain variable region (VH) comprising a complementarity determining region (CDR) H1 comprising the amino acid sequence represented by SEQ ID NO: 9, a complementarity determining region (CDR) H2 comprising the amino acid sequence represented by SEQ ID NO: 10, a complementarity determining region (CDR) H3 comprising the amino acid sequence represented by SEQ ID NO: 11, and an antibody light chain variable region (VL) comprising a complementarity determining region (CDR) L1 comprising the amino acid sequence represented by SEQ ID NO: 12, a complementarity determining region (CDR) L2 comprising the amino acid sequence represented by SEQ ID NO: 13, and a complementarity determining region (CDR) L3 comprising the amino acid sequence represented by SEQ ID NO: 14;
iii) an antibody comprising an antibody heavy chain variable region (VH) comprising a complementarity determining region (CDR) H1 comprising the amino acid sequence represented by SEQ ID NO: 17, a complementarity determining region (CDR) H2 comprising the amino acid sequence represented by SEQ ID NO: 18, a complementarity determining region (CDR) H3 comprising the amino acid sequence represented by SEQ ID NO: 19, and an antibody light chain variable region (VL) comprising a complementarity determining region (CDR) L1 comprising the amino acid sequence represented by SEQ ID NO: 20, a complementarity determining region (CDR) L2 comprising the amino acid sequence represented by SEQ ID NO: 21, and a complementarity determining region (CDR) L3 comprising the amino acid sequence represented by SEQ ID NO: 22; and
iv) an antibody comprising an antibody heavy chain variable region (VH) comprising a complementarity determining region (CDR) H1 comprising the amino acid sequence represented by SEQ ID NO: 25, a complementarity determining region (CDR) H2 comprising the amino acid sequence represented by SEQ ID NO: 26, a complementarity determining region (CDR) H3 comprising the amino acid sequence represented by SEQ ID NO: 27, and an antibody light chain variable region (VL) comprising a complementarity determining region (CDR) L1 comprising the amino acid sequence represented by SEQ ID NO: 28, a complementarity determining region (CDR) L2 comprising the amino acid sequence represented by SEQ ID NO: 29, and a complementarity determining region (CDR) L3 comprising the amino acid sequence represented by SEQ ID NO: 30.

2. The antibody or fragment thereof that binds to human CTLA-4 protein according to claim 1, wherein the antibody is selected from the group consisting of:
i) an antibody comprising a heavy chain variable region comprising the amino acid sequence from the 1st to 116th of SEQ ID NO: 7 and a light chain variable region comprising the amino acid sequence from the 1st to 110th of SEQ ID NO: 8;
ii) an antibody comprising a heavy chain variable region comprising the amino acid sequence from the 1st to 116th of SEQ ID NO: 15 and a light chain variable region comprising the amino acid sequence from the 1st to 109th of SEQ ID NO: 16;
iii) an antibody comprising a heavy chain variable region comprising the amino acid sequence from the 1st to 121st of SEQ ID NO: 23 and a light chain variable region comprising the amino acid sequence from the 1st to 110th of SEQ ID NO: 24; and
iv) an antibody comprising a heavy chain variable region comprising the amino acid sequence from the 1st to 116th of SEQ ID NO: 31 and a light chain variable region comprising the amino acid sequence from the 1st to 110th of SEQ ID NO: 32.

3. The antibody or fragment thereof that binds to human CTLA-4 protein according to claim 1, wherein the antibody is selected from the group consisting of:
i) an antibody comprising a heavy chain consisting of SEQ ID NO: 7 and a light chain consisting of SEQ ID NO: 8;
ii) an antibody comprising a heavy chain consisting of SEQ ID NO: 15 and a light chain consisting of SEQ ID NO: 16;
iii) an antibody comprising a heavy chain consisting of SEQ ID NO: 23 and a light chain consisting of SEQ ID NO: 24; and
iv) an antibody comprising a heavy chain consisting of SEQ ID NO: 31 and a light chain consisting of SEQ ID NO: 32.

4. The antibody or fragment thereof according to claim 1, wherein the fragment is selected from the group consisting of diabody, Fab, Fab', F(ab)₂, F(ab')₂, Fv and scFv.

5. A polynucleotide encoding the antibody or fragment thereof according to claim 1.

6. A recombinant vector comprising the polynucleotide according to claim 5.

7. A transformed cell line comprising the vector according to claim 6.

8. A method for producing an antibody or a fragment thereof that binds to human CTLA-4 protein, comprising culturing the cell line according to claim 7 under conditions in which the polynucleotide is expressed to produce a polypeptide comprising a light chain and heavy chain variable region; and recovering the polypeptide from the cell line or culture medium in which the cell line was cultured.

9. A pharmaceutical composition for preventing or treating cancer comprising the antibody or fragment thereof according to claim 1 as an active ingredient.

10. The pharmaceutical composition according to claim 9, wherein the cancer is selected from the group consisting of breast cancer, colorectal cancer, head and neck cancer, colon cancer, skin cancer, pancreatic cancer, lung cancer, gastric cancer, ovarian cancer, prostate cancer, bladder cancer, urethral cancer, liver cancer, kidney cancer, clear cell sarcoma, melanoma, brain and spinal cord tumors, brain cancer, thymoma, mesothelioma, esophageal cancer, bile duct cancer, testicular cancer, germ cell tumor, thyroid cancer, parathyroid cancer, cervical cancer, endometrial cancer, lymphoma, myelodysplastic syndromes (MDS), myelofibrosis, acute leukemia, chronic leukemia, multiple myeloma, Hodgkin's disease, endocrine cancer, and sarcoma.

11. Use of the antibody or fragment thereof according to claim 1 for manufacturing a pharmaceutical composition for treating cancer.

12. A method for treating cancer comprising administering an effective amount of a pharmaceutical composition comprising the antibody or fragment thereof according to claim 1 as an active ingredient to a subject in need thereof.
